# FASCICULE DE BREVET EUROPEEN

(11) **EP 3 031 454 B1**
(45) Date de publication et mention de la délivrance du brevet: **18.11.2020**
(21) Numéro de dépôt: 16153926.7
(22) Date de dépôt: 28.02.2013
(51) Int. Cl.: A61K 9/70, A61K 31/465

(54) **DISPOSITIF TRANSDERMIQUE COMPRENANT DES MICRO-PARTICULES POREUSES**
TRANSDERMALE VORRICHTUNG, DIE PORÖSE MIKROPARTIKEL UMFASST
TRANSDERMAL DEVICE COMPRISING POROUS MICROPARTICLES

(30) Priorité: 02.03.2012 FR 1251940
(43) Date de publication de la demande: 15.06.2016
(62) Demande divisionnaire de: 13707357.3
(73) Titulaire: PIERRE FABRE MEDICAMENT, 92100 Boulogne-Billancourt (FR)
(72) Inventeur: RIBEIRO DOS SANTOS, Isabel, 31600 EAUNES (FR); SOURNAC, Michel, 31400 TOULOUSE (FR)
(74) Mandataire: Regimbeau

(56) Documents cités:
- WO-A1-95/01766
- US-A1- 2011 066 120
- MARYKA QUIK ET AL: "Nicotine and Nicotinic Receptors; Relevance to Parkinson's Disease", NEUROTOXICOLOGY,, vol. 23, no. 4-5, 1 octobre 2002 (2002-10-01), pages 581-594, XP055062376,
- Viral Shaha ET AL: "Microsponge drug delivery: A Review", Int. J. Res. Pharm. Sci., 1 janvier 2010 (2010-01-01), pages 212-218, XP055041141, Extrait de l'Internet: URL:http://ijrps.pharmascope.org/downloads /Volume 1/Issue 2/55945.pdf [extrait le 2012-10-16]
- PARTHIBAN, K.G. ET AL: "Microsponge role in novel drug delivery system", INTERNATIONAL JOURNAL OF PHARMACEUTICAL RESEARCH AND DEVELOPMENT, vol. 3, no. 4, juin 2011 (2011-06), pages 117-125, XP002685345,

## Description

La présente invention concerne le domaine des micro-particules polymère poreuses et leur utilisation associée à la nicotine dans un système de délivrance transdermique. La présente invention porte, en outre, sur un dispositif transdermique contenant lesdites microparticules poreuses et son utilisation en tant que médicament.

Plusieurs familles de polymères solides (polyamides, polyacrylonitriles, polyacrylique, chlorure de polyvinylidène, copolymères réticulés, chlorure de vinyle, etc.) sont connues pour leurs usages cosmétiques comme agents structurants, texturant, absorbeurs de sébum, matifiants. Dans chacun de ces cas, il s'agit de formuler un produit cosmétique pour faciliter son application, lui conférer une propriété organoleptique particulière ou permettre la délivrance locale d'un principe actif.

La technologie telle que décrite dans le brevet d'Advanced Polymer Systems Inc. permet l'obtention de particules (ou microparticules) poreuses solides par polymérisation (par exemple US 5,145,675 et US 5,156,843)

Les polymères selon ces brevets d'Advanced Polymer Systems Inc. sont notamment constitués d'unités monomères tels que le styrène, le divinylbenzène, le méthylméthacrylate, l'éthylèneglycol diméthacrylate, le 4-vinylpyridine, le laurylméthacrylate, le méthacrylate d'allyle, le glycol diméthacrylate. Un polymère particulier à base de copolymères réticulés méthylméthacrylate - éthylène glycol diméthacrylate sous forme de microparticules est commercialisé sous le nom de Microsponge®.

Ces particules poreuses ont une taille unitaire pouvant varier entre 10 et 400 µm, elles peuvent ainsi piéger de manière physique de fortes quantités de principes actifs puis les libérer de manière plus ou moins contrôlée dans le temps. Par exemple, une sphère typique de 25µm de Microsponge® peut avoir jusqu'à 250,000 pores ce qui conduit à un volume total de pores proche de 1ml/g. Plus souvent, cette capacité est caractérisée par une mesure de surface spécifique exprimée en m²/g de produit. Du fait de leur taille, ces microparticules ne peuvent pas franchir le stratum corneum. La libération de ces actifs se fait par simple diffusion ou via plusieurs stimuli (friction mécanique - hydratation - pH - température) qui ont été décrits.

Cette technologie est plus particulièrement adaptée à la formulation de produits médicamenteux topiques (crèmes, gels, lotions, poudres, savons, etc.) ou de soins esthétiques. Dans les deux cas, les Microsponge® permettent d'améliorer la tolérance cutanée. Par exemple, en dermo-cosmétique médicale, plusieurs produits sont commercialisés sous forme de gels ou de crèmes, à base de peroxyde de benzoyle (NeoBenz®), de trétinoin (Retin-A® Micro), de fluorouracil (Carac® 0,5%) ou de rétinol et hydroquinone (EpiQuin® Micro). Pour chacun de ces médicaments, l'avantage est aussi de pouvoir maximiser la présence du principe actif à la surface de la peau ou dans l'épiderme, tout en réduisant son passage dans le derme.

Des compositions faisant appel à la technologie Microsponge® ont été rapportées et on peut citer à ce titre US 5,145,675 et EP 0306236 qui décrivent comment délivrer localement sur la peau et de manière prolongée et contrôlée certaines substances comme des écrans solaires, des repellants, des stéroïdes cutanés comme le fluocinonide ou le peroxyde de benzoyle, des anti-irritants, des anti-chutes capillaire comme le minoxidil ou des vitamines, essentiellement. Les supports poreux mis en oeuvre sont ceux décrits par exemple dans WO 88/01164, soit un copolymère de styrène et divinylbenzène ou un copolymère de méthylméthacrylate - éthylène glycol diméthacrylate ou encore un copolymère de 4-vinylpyridine et de diméthacrylate d'éthylèneglycol. Ces microparticules sont obtenues dans une première étape par polymérisation en suspension en présence d'un agent porogène, suivie par une étape d'extraction de ce porogène avant l'incorporation finale du principe actif.

La technologie Microsponge® n'est pas utilisée dans la formulation de dispositifs transdermiques.

Le système de délivrance transdermique, ou dispositif transdermique, ou patch, utilise la voie cutanée pour assurer l'administration directe du principe actif dans le réseau sanguin sans passer par le système digestif, comme c'est le cas des comprimés ou des sirops.

Un système de délivrance transdermique est un dispositif adhésif qui permet d'administrer, dans un but médical, une substance active à travers la peau.

Ce mode de fonctionnement, outre le confort d'utilisation qu'il offre, présente de nombreux avantages.

En premier lieu, il élimine le risque de dégradation du médicament par les sucs gastriques, la métabolisation hépatique ou la voie intestinale. Il est particulièrement intéressant de présenter sous forme de patch des molécules que le foie tend à retenir. En effet, s'il les élimine, ou les stocke, lors de leur administration par voie orale, elles se trouvent dévoyées de leur fonction thérapeutique et perdent toute efficacité. Parallèlement à cela, le patch élimine le risque de troubles digestifs liés à l'ingestion d'une substance. Enfin, contrairement aux autres dispositifs qui entraînent des pics de concentration dans le système sanguin, le patch libère les substances actives qu'il contient avec une bonne régularité au fil des heures. En conséquence, il peut maintenir une concentration relativement constante du médicament dans l'organisme.

Il réduit ainsi au maximum les effets secondaires (irritation digestive par exemple), la métabolisation hépatique (effet de premier passage) et la dégradation intestinale des médicaments en cause. Aujourd'hui, un nombre significatif de patchs sont disponibles pour différents traitements. Dans le cas du sevrage tabagique, la nicotine est notamment administrée à l'aide de patchs..

Il existe plusieurs types de patchs nicotiniques pouvant être catégorisés (dans « Topical Drug Bioavailability, Bioequivalence and Penetration, V.P Shah et H.I Maibach, édité par Springer 1993, page 53) selon le mécanisme de libération du principe actif et le design du patch. Parmi les six catégories répertoriées, les patchs à base de nicotine sont ceux des catégories II, IVa et IVc, isolément ou sous la forme d'une combinaison. En incorporant directement la nicotine dans l'adhésif, le patch de type IVa comme celui de la présente invention présente l'avantage d'avoir une faible épaisseur et de pouvoir être découpé à titre exceptionnel grâce à son homogénéité de répartition du principe actif sur l'ensemble de sa surface, contrairement par exemple aux patchs de type II comportant une zone de dépôt.

L'absorption transcutanée d'une substance répond à des règles bien précises que mettent à profit les patchs pour maîtriser le dosage tout au long de la durée d'utilisation. Le phénomène correspond à la migration de la substance considérée de la surface de la peau jusqu'à la première couche de capillaires sanguins. Mais les molécules capables de pénétrer l'épiderme sont rares. À l'heure actuelle, on n'en connaît qu'une douzaine qui sont administrées par patch.

En effet, leurs caractéristiques physicochimiques doivent être telles que la peau ne constitue pas un obstacle infranchissable - entre autres, leur masse moléculaire doit être inférieure à 500 daltons, et leur point de fusion doit être au-dessous de 100 °C. Par ailleurs, les substances candidates au patch doivent bien sûr être tolérées par la peau, afin d'éviter toute réaction allergique.

Les propriétés physicochimiques de la nicotine sont problématiques pour une administration transcutanée, en effet, la nicotine sous forme de base est une substance huileuse qui pénètre la surface de la peau jusqu'à la première couche de capillaires sanguins extrêmement rapidement. De plus, il s'agit d'une substance très toxique. Il ne faut donc pas que, dès l'apposition du patch, l'ensemble de la nicotine qu'il contient transite dans l'organisme en quelques minutes : sa concentration dans le sang doit rester constante, à une dose thérapeutique, durant une douzaine d'heures. Pour cela, la nicotine est formulée dans un patch dont la composition régule le transfert de la nicotine. De plus, la loi de diffusion de Fick permet de déterminer un taux d'administration de la nicotine, exprimé en microgrammes par heure et par centimètre carré de patch, en fonction de la composition du patch qui lui est associé. On contrôle ainsi très précisément la dose injectée et la constance de son administration au fil des heures. Généralement, après un temps de latence correspondant au délai indispensable pour que le principe actif traverse l'épiderme et atteigne la circulation sanguine, sa concentration croît rapidement de manière exponentielle jusqu'à atteindre un état d'équilibre correspondant à la valeur fixée par le patch. Cette concentration se maintient jusqu'au retrait du patch ou jusqu'à l'épuisement de sa réserve de substance active.

Par ailleurs, avec un patch, on peut très facilement interrompre le traitement en cas d'intolérance de la part du patient. Il suffit de le décoller pour mettre fin à l'apport de médicament, ou de substance palliative dans le cas de la nicotine. L'atout principal du patch reste son confort d'utilisation et la délivrance régulée dans le temps du principe actif.

Devant les difficultés à contrôler la cinétique de libération de la nicotine, plusieurs dispositifs contenant des membranes de contrôle ou des microparticules solides ont été décrits. On peut citer par exemple dans EP 708627, un patch nicotinique comprenant une couche support, une couche matricielle composée d'un mélange entre la nicotine et un polymère, le dit patch étant caractérisé par un flux moyen de nicotine supérieur à 50 µg/cm²/hr. Les compositions du brevet EP 708627 sont caractérisées par l'emploi d'adhésif de type silicone et de particules solides de type silice.

D'autres compositions de patch nicotine sont également connues, comme US 5,725,876 décrivant un patch nicotine matriciel de type acrylique comportant des argiles de manière à améliorer la cohésion du dispositif. Pour ces deux exemples, les microparticules solides décrites ne sont pas des polymères poreux comme celles de la présente invention. Le document US 2011/066120 A1 décrit un dispositif transdermique auto-adhésif multicouche, où la première et/ou la troisième couche adhésive peuvent comprendre des particules de silice microporeux contenant un principe actif dans leurs pores pour le contrôle de la vitesse de pénétration dans la peau.

La demanderesse a cherché à concevoir un dispositif transdermique auto-adhésif de substitution aux dispositifs déjà existants. La demanderesse a cherché à concevoir un dispositif transdermique capable de contrôler la libération de nicotine tout en conservant les attributs d'un patch matriciel de type « drug in adhesive » tels que faible épaisseur, taille réduite, ainsi qu'un procédé de fabrication plus simple et moins onéreux que ceux déjà connus dans l'art antérieur.

La demanderesse a cherché à résoudre un certain nombre de problèmes techniques auxquels ne répondaient pas les dispositifs transdermiques auto-adhésif existants :
- obtenir un patch caractérisé par une répartition homogène de nicotine dans la matrice adhésive, ce qui autorise de manière exceptionnelle la découpe du patch.
- maîtriser la plastification induite par la présence de nicotine ce qui revient à maintenir la cohésion de la matrice adhésive.
- avoir un meilleur confort grâce à un patch moins épais, plus souple et donc plus confortable à utiliser.

De plus un certain nombre de contraintes sont préférentiellement à respecter concernant le dispositif transdermique à obtenir :
- contrôler la libération de nicotine (ad minima, éviter une libération trop rapide, synonyme d'effets secondaires notamment addiction, probablement cardiaque et de type cutané) avec un nouveau support matriciel.
- avoir de bonnes qualités d'adhésion et de tolérance cutanée.
- obtenir un patch dont le vieillissement est sans conséquences sur les caractéristiques physico chimiques initiales du produit.
- sélectionner des composants compatibles avec la nicotine.
- obtenir un patch transparent (cela permet d'observer éventuellement un phénomène d'irritation ou de sensibilisation locale

De façon surprenante et inattendue, la demanderesse a montré que l'utilisation de microparticules poreuses de polymères solides chargées avec de la nicotine dans un système de délivrance transdermique présentait de nombreux avantages, particulièrement au niveau de la libération contrôlée de la nicotine et de la répartition homogène du principe actif dans la matrice adhésive. En outre l'utilisation de ces microparticules permet, de façon tout à fait inattendue, d'augmenter la cohésion du patch (permettant notamment de réduire le risque de fuite et la quantité de résidus de matrice restant sur la peau après retrait du patch) et de réduire l'agressivité de l'adhésion du patch.

La présente invention a également pour objet un procédé de préparation d'un dispositif transdermique selon l'invention.

L'utilisation de polymères solides microporeux notamment de ceux constitués d'unités monomères tels que le styrène, le divinylbenzène, le méthylméthacrylate, l'éthylèneglycol diméthacrylate, le 4-vinylpyridine, le laurylméthacrylate, le méthacrylate d'allyle et/ou le glycol diméthacrylate a montré une très bonne dispersion au sein de l'adhésif et une meilleure compatibilité avec la nicotine (pas de dégradation du principe actif, pas de coloration observée).

En outre, le procédé de fabrication de la composition selon la présente invention est plus simple que celui lié à la fabrication de certains dispositifs transdermiques auto-adhésifs existants comme le patch Nicotinell®, notamment décrit dans Topical Drug Bioavailability, Bioequivalence and Penetration, (V.P Shah et H.I Maibach, édité par Springer 1993, page 53) car contrairement à ce dernier, le patch de nicotine selon la présente invention est un patch monocouche qui ne nécessite qu'un seul passage dans une ligne d'enduction /séchage.

Par ailleurs, l'objet de la présente invention se différencie de certains dispositifs transdermiques pour la libération de nicotine existant jusqu'ici. En effet, ces derniers ne peuvent pas être découpés, et de plus, avec le temps, la répartition en nicotine dans le patch devient hétérogène. Par effet de maturation, il a pu être mis en évidence sur certains des patchs de l'art antérieur une répartition décroissante et concentrique en nicotine du centre vers les zones périphériques.

Un patch transdermique selon l'invention contient une répartition homogène de la nicotine dans toute sa partie matricielle. Cette caractéristique permet une meilleure utilisation du dispositif par les patients, notamment à travers une plus grande finesse du patch, mais également à travers la possibilité de découper le patch afin de diminuer la dose absorbée et ce, sans altérer les fonctions de celui-ci.

Les caractéristiques et les avantages des compositions selon la présente invention apparaissent à la lumière de la description ci après.

Description des figures:
Figures 1 et 2 : Cinétiques de perméation *in vitro* montrant l'effet de la présence de polymère solide microporeux (Microsponge®) dans la composition
Figure 3 : Compositions monocouches adhésives et résultats de perméation en fonction de l'adhésif utilisé.
Figure 4 : Cinétiques de perméation *in vitro* montrant l'équivalence de l'effet de la présence de polymère solide microporeux Microsponge® ou Polytrap® dans la composition selon l'invention.

La présente invention porte donc sur un dispositif transdermique auto-adhésif, caractérisé en ce qu'il comprend l'association d'une couche support et d'une couche matricielle auto-adhésive, et un film protecteur détachable, ladite couche matricielle auto-adhésive comprenant, par rapport au poids total de la couche matricielle auto-adhésive :
a. 65 à 93% en poids d'au moins un polymère auto-adhésif choisi dans le groupe comprenant les polymères de type acrylique ou acrylate, les polymères de type silicone, les polymères de type acétate de vinyle, les gommes naturelles ou synthétiques leurs copolymères et leurs mélanges ;
b. 2 à 15% en poids d'au moins un polymère solide microporeux pouvant contenir un principe actif, le dit polymère étant constitué d'unités monomères sélectionnées dans le groupe de monomères comprenant le laurylméthacrylate, et le glycol diméthacrylate et
c. 5 à 20% en poids de principe actif.

Par « association », on entend que les couches du dispositif selon l'invention sont directement en contact les unes avec les autres et qu'une fois assemblées, elles forment un tout qui ne peut se défaire facilement et en particulier qui ne peut se défaire sans intervention et volonté humaine.

Préférentiellement, le patch selon l'invention sera du type monocouche adhésive.

Par « couche support selon l'invention », on entend toute couche support classiquement utilisée dans le domaine des patchs. Préférentiellement, la couche support est un film multicouche dont au moins une couche est une couche polyester et au moins une autre couche est une couche à base de polyéthylène ou d'éthylène de vinyle acétate. Préférentiellement, la couche support est transparente.

Par « film protecteur détachable », on entend tous les films protecteurs détachables capables de protéger la matrice du dispositif avant l'utilisation du dispositif. Ces films classiquement utilisés dans le domaine des dispositifs transdermique sont bien connus de l'homme du métier. Préférentiellement, ce film est en polyester siliconé.

La couche matricielle selon l'invention est auto-adhésive. Par « auto-adhésive », on entend que la couche matricielle est apte à maintenir fixé le dispositif selon l'invention sur un support, par exemple la peau, et ce, de façon stable, sans qu'il y ait besoin de l'intervention d'autres moyens de fixation. Préférentiellement, par « auto-adhésif », on entend que la couche matricielle permet au dispositif d'adhérer à la peau pendant au moins 12 heures, préférentiellement de 12 à 48 heures environ, encore préférentiellement 24 heures environ.

La couche matricielle selon l'invention comprend de 65 à 93%, préférentiellement entre 75 et 85% en poids d'au moins un polymère auto-adhésif choisi dans le groupe comprenant les polymères de type acrylique ou acrylate, les polymères de type silicone, les polymères de type acétate de vinyle, les gommes naturelles ou synthétiques, leurs copolymères et leurs mélanges.

Par gomme synthétique on entend notamment au sens de la présente invention les gommes sélectionnées dans la famille des élastomères, par exemple le styrène, le styrène- isoprène-styrène (SIS), le polystyrène-butadiène-styrène (SBS) ou le styrène-éthylène/butylène-styrène (SEBS). Par gomme naturelle on entend toutes les sortes de gommes naturelles telles que la gomme de carraghénane ou la gomme de guar par exemple.

Préférentiellement, la couche matricielle comprend au moins un polymère de type acrylique ou acrylate.

Le polymère de type acrylique ou acrylate selon l'invention sera préférentiellement constitué par des monomères sélectionnés dans le groupe comprenant ou consistant en l'acétate de vinyle, le 2-éthylhexyl acrylate, l'acrylate de butyle, l'acide acrylique, le méthyle méthacrylate, le méthyle acrylate, le ter octyl acrylamide, le 2-hydroxy éthyle acrylate, le glycidyle méthacrylate, ou leurs mélanges, encore préférentiellement dans le groupe comprenant ou consistant en en l'acide acrylique, l'acrylate de butyle, le 2-éthylhéxyl acrylate, l'acétate de vinyle, et leurs mélanges.

Dans un mode de réalisation, le polymère auto-adhésif selon l'invention est un copolymère acrylate-acétate de vinyle.

La couche matricielle selon l'invention comprend, en outre, de 2 à 15%, préférentiellement de 5 à 12% en poids par rapport au poids total de la couche matricielle auto-adhésive d'au moins un polymère solide microporeux pouvant contenir un principe actif.

Par « pouvant contenir un principe actif » selon l'invention on entend que le polymère solide microporeux peut être chargé de principe actif par adsorption, le principe actif est donc associé au polymère. Préférentiellement, le polymère solide microporeux selon l'invention pourra être chargé de 15% à plus de 400%, préférentiellement de plus de 50 % par exemple entre 50 et 150%, encore par exemple autour de 100% ou de 140% en poids de principe actif par rapport au poids du polymère solide microporeux libre. Les polymères solides microporeux selon l'invention sont préférentiellement chargés par simple contact (adsorption) avec le principe actif par mélange.

Le polymère solide microporeux pouvant contenir un principe actif selon l'invention sera préférentiellement des microparticules poreuses solides de type polymère ou copolymère réticulé (polymère solide microporeux).

Préférentiellement, le dit polymère solide microporeux est constitué d'unités monomères sélectionnées dans le groupe de monomères comprenant ou consistant en le laurylméthacrylate, et le glycol diméthacrylate .

Le polymère solide microporeux est constitué d'unités de monomères de laurylméthacrylate et de glycol diméthylméthacrylate. Un tel polymère peut notamment être obtenu de façon commerciale sous la marque Polytrap®. Préférentiellement, le dispositif transdermique auto-adhésif selon l'invention ne contient pas d'autre polymère solide microporeux que le polymère solide microporeux constitué d'unités de monomères de laurylméthacrylate et de glycol diméthylméthacrylate

Préférentiellement, le polymère solide microporeux selon l'invention se présente sous la forme de microparticules de taille moyenne comprise entre 1 µm et 100 µm, préférentiellement entre 5 µm et 50 µm, encore préférentiellement entre 15 µm et 30 µm. La mesure de la taille moyenne peut notamment être réalisée par un appareil de type granulomètre laser, par exemple le Malvern Mastersizer 2000 qui permet, en particulier, de mesurer des particules allant de 0,2 µm à 2000 µm en voie sèche.

Dans un mode de réalisation de l'invention, le polymère solide microporeux selon l'invention a un volume poreux total compris entre 0,5 et 3 ml/g, préférentiellement entre 1 et 2 ml/g.

Dans un autre mode de réalisation, le dit polymère microporeux a un volume poreux granulaire compris entre 0,2 et 3ml/g, préférentiellement entre 0,5 à 1,5 ml/g, toujours préférentiellement entre 0,7 et 1,0 ml/g.

Le volume poreux total et le volume poreux granulaire pourront notamment être mesurés par un porosimètre à mercure, par exemple l'Autopore IV (Micromeritics). Ces porosimètres permettent en particulier de réaliser une intrusion de mercure à pression constante et ainsi de pouvoir déterminer le volume poreux total et un volume poreux granulaire pour des polymères solides microporeux. A basse pression (par exemple de 3,45 KPa à 186 KPa), les volumes d'intrusion obtenus correspondent au tassement de la poudre et à la porosité totale (inter granulaire et granulaire). A haute pression (par exemple de 186 KPa à 20,7 MPa), les valeurs d'intrusion de mercure observées correspondent à la porosité granulaire.

Selon une caractéristique avantageuse de la présente invention, le polymère solide microporeux, qui se présente avantageusement sous forme de microparticules, est distribué de manière homogène au sein de la masse de polymère auto-adhésif. Selon une autre caractéristique de l'invention, le principe actif est adsorbé de manière homogène dans la masse de polymère solide microporeux. Ainsi, il en résulte une distribution régulière dans la couche matricielle auto-adhésive du dispositif de l'invention et, partant, une libération régulière du principe actif.

Préférentiellement, le dispositif selon l'invention est caractérisé en ce que le grammage de la couche matricielle autoadhésive est compris entre 50 et 300 g/m², préférentiellement entre 100 et 200 g/m².

Par principe actif selon l'invention on entend n'importe quel principe actif ou n'importe quel mélange de principes actifs administrable par un dispositif selon l'invention. Dans un mode de réalisation le principe actif pourra être sous forme liquide, préférentiellement sous forme d'un liquide huileux. Par exemple, le principe actif selon l'invention est a nicotine. Dans un mode de réalisation le principe actif est sélectionné dans le groupe comprenant ou consistant en la nicotine. Le principe actif selon l'invention est avantageusement un principe actif dont il faut freiner/contrôler la libération tel que par exemple la nicotine Préférentiellement, le principe actif selon l'invention est la nicotine, encore préférentiellement le dispositif selon l'invention ne comprend pas d'autre principe actif que la nicotine.

Le principe actif pourra être chargé dans le polymère solide microporeux sous forme liquide. Le principe actif, notamment la nicotine sera préférentiellement chargé sous forme de base ou sous forme d'une solution de l'un de ses sels, encore préférentiellement sous forme de base. La quantité de principe actif chargé dans les particules dépendra notamment de la quantité souhaitée de principe actif relargué par le dispositif transdermique selon l'invention et du temps de relarguage souhaité. Par exemple, le dispositif selon l'invention comprenant de la nicotine pourra comprendre entre 10 et 100 mg de nicotine, préférentiellement entre 15 et 90 mg de nicotine, par exemple 15, 30, 45 ou 90 mg de nicotine.

Les inventeurs ont constaté que la libération de la nicotine était particulièrement bien contrôlée lorsque le polymère solide microporeux est tel que défini dans la présente invention, préférentiellement, un copolymère de méthylméthacrylate et éthylèneglycol diméthacrylate, et que la matrice adhésive à un grammage entre 50 et 300 g/m², préférentiellement entre 100 et 200 g/m².

La vitesse de libération de la nicotine par les polymères solides microporeux dépendra notamment de la nature du polymère et de la taille des pores. Préférentiellement, les dispositifs selon l'invention permettent la libération de la nicotine par un flux moyen à partir de la couche matricielle compris entre 5 et 100 µg/cm²/h, dans un mode de réalisation entre 10 µg/cm²/h et 50 µg/cm²/h, pendant un intervalle de temps compris entre 1 et 24h. La vitesse de libération peut être mesurée par n'importe quelle technique de perméation connue de l'homme du métier, notamment via une cinétique de perméation par exemple sur peau abdominale de souris nude disposée à la surface d'une cellule en verre (« cellule de Franz », tel que notamment décrit dans l'exemple 3).

La couche matricielle selon l'invention peut, en outre, comprendre d'autres excipients ou composants tels que des antioxydants par exemple. Dans un mode de réalisation, la couche matricielle auto-adhésive comprend au moins un agent antioxydant notamment choisi dans le groupe comprenant ou consistant en le butyle hydroxy toluène (BHT), le butyle hydroxy anisole (BHA), le palmitate d'ascorbyle, l'alpha tocophérol et ses esters, l'acide citrique, le gallate de propyle et leurs mélanges, préférentiellement le BHT. L'agent antioxydant est alors préférentiellement présent dans ladite couche matricielle auto-adhésive dans une proportion comprise entre 0,01 et 1% en poids par rapport au poids total de ladite couche matricielle auto-adhésive.

Dans un autre mode de réalisation, le dispositif transdermique auto-adhésif selon l'invention est caractérisé en ce qu'il consiste en l'association d'une couche support et d'une couche matricielle auto-adhésive, et un film protecteur détachable, ladite couche matricielle auto-adhésive consistant en, par rapport au poids total de la couche matricielle auto-adhésive :
a. 65 à 93% en poids d'au moins un polymère auto-adhésif choisi dans le groupe comprenant les polymères de type acrylique, les polymères de type silicone, les polymères de type acétate de vinyle, les gommes naturelles ou synthétiques, leurs copolymères et leurs mélanges ;
b. 2 à 15% en poids d'au moins un polymère solide microporeux pouvant contenir un principe actif, le dit polymère étant constitué d'unités monomères sélectionnées dans le groupe de monomères comprenant le laurylméthacrylate, et le glycol dimethacrylate;
c. 5 à 20% en poids de nicotine en tant que principe actif.
d. optionnellement de 0,1 à 1 % d'un agent anti-oxydant ; et

De préférence, le patch selon l'invention sera transparent. Par « transparent », on entend qu'il permet de suffisamment distinguer la peau à travers le patch pour identifier rapidement toute éventuelle intolérance cutanée.

La présente invention porte en outre sur un procédé de préparation d'un dispositif transdermique selon l'invention caractérisé en ce qu'il comprend ou consiste en les étapes suivantes :
a. Mélanger le polymère auto-adhésif, le polymère solide microporeux, le principe actif, préférentiellement la nicotine et optionnellement, l'agent oxydant jusqu'à obtention d'un mélange homogène ;
b. Enduire le mélange homogène obtenu à l'étape a. sur un film protecteur détachable ;
c. Sécher progressivement le mélange enduit;
d. Contrecoller le produit obtenu à l'étape c. sur une couche support ; et
e. Optionnellement, découper le produit obtenu à l'étape d. de manière à obtenir des dispositifs transdermiques ou patchs de taille désirée.

Par mélanger le polymère auto-adhésif et le polymère solide microporeux selon l'invention « jusqu'à obtention d'un mélange homogène selon la présente invention », on entend que le polymère solide microporeux doit être réparti de façon uniforme dans le polymère autoadhésif. Cette répartition peut notamment être évaluée par observation visuelle (absence d'agrégats).

La nicotine peut ou non être mélangée au support microporeux selon l'invention avant l'étape a. Dans un mode de réalisation préféré, la nicotine n'est pas mélangée préalablement au support microporeux avant l'étape a., la nicotine, le support microporeux et le polymère autoadhésif étant alors mis en contact indépendant et de façon concomitante à l'étape a.

Préférentiellement, l'étape b. d'enduction est réalisée à une température comprise entre 30°C et 50°C.

Par «séchage progressif », on entend la mise en place d'un gradient de températures. Il est préférable que le séchage se fasse progressivement afin d'éviter un phénomène de croutage. Par exemple, le gradient de température pourra être un gradient de 5 à 20°C dans une gamme de température allant de 30°C à 80°C, préférentiellement de 40 à 60 °C.

Après enduction et séchage, le produit obtenu à l'étape c. est contrecollé sur une couche support selon l'invention. L'opération de contrecollage peut être réalisée par toutes les méthodes bien connues de l'homme du métier.

La couche support enduite obtenue à l'étape d. du procédé selon l'invention pourra ensuite éventuellement être découpée à la taille désirée. Par exemple, la couche support pourra être découpée en dispositifs transdermiques ou patch (ou unités) entre 5 cm² et 80 cm², par exemple de 10cm², 20 cm², 30 cm² ou 60 cm².

Le dispositif transdermique selon l'invention pourra être conditionné dans un emballage sous atmosphère protectrice (inerté), préférentiellement sous azote, en particulier lorsque le patch selon l'invention ne contient pas d'agent anti-oxydant. L'inertage des conditionnements (emballages) de patch est bien connu de l'homme du métier et pourra être réalisé par toutes les méthodes connues.

La présente invention porte, en outre, sur un dispositif transdermique auto-adhésif selon l'invention pour son utilisation en tant que médicament.

En particulier, la présente invention porte sur un dispositif transdermique auto-adhésif selon l'invention, préférentiellement un dispositif transdermique selon l'invention contenant de la nicotine, pour son utilisation dans le traitement de la dépendance à la nicotine en particulier afin de facilité le sevrage tabagique.

Dans un autre mode de réalisation, la présente invention porte sur un dispositif transdermique auto-adhésif, préférentiellement un dispositif transdermique selon l'invention contenant de la nicotine, pour son utilisation dans le traitement des maladies neurodégénératives telles que notamment la maladie de Parkinson ou la maladie d'Alzheimer.

La présente invention est illustrée par les exemples ci-dessous qui ne limitent pas sa portée.

### EXEMPLES :

### Exemple 1 : Caractérisation des supports d'adsorption

### Exemple 1A : Tests de capacité d'adsorption (tableau 1) :

Des tests comparatifs destinés à mesurer la capacité d'adsorption de la nicotine par différents solides microporeux ont été effectués. Les tests ont consisté à déterminer la quantité maximale de nicotine pouvant être adsorbée sur les supports microporeux selon le mode opératoire suivant :
- pesée du support microporeux, entre 0,3 et 2,0g selon sa densité pour disposer d'un volume identique,
- ajout de la nicotine de manière itérative par pesée de 0,1g environ,
- imprégnation de la nicotine sur le support à l'aide d'une spatule après chaque ajout de nicotine ; l'aspect du mélange est alors relevé.

La capacité maximale d'adsorption est ainsi déterminée : il s'agit de la quantité de nicotine adsorbée par le support microporeux exprimé en gramme de nicotine par gramme de support, sans observation de suintement de nicotine.

On a obtenu les résultats suivants : Tableau 1 :

| Type de support | Nom commercial | Capacité d'adsorption (quantité de nicotine en gr/1gr de sup | port) Commentaires - Aspect visuel |
|---|---|---|---|
| Polymère réticulé MMA/GDMA | Microsponge® | 1,40 | Gonflement important des particules; maintien d'une couleur jaune clair |
| Silice | Silica Shells | 20,70 | Particules très petites formant des grappes une fois imprégnées |
| Silice | MSS-500/3H4® | 3,00 | Homogénéisation difficile avec formation de grumeaux |
| Silice pyrogénée densifiée | Aerosil®V200 | 2,75 | La nicotine p énétre mais ne s'étale pas ; les particules forment des amas irréguliers |
| Polyuréthane/silice | Microsphères BPD 800® | 1,05 | Gonflement important des particules; maintien d'une couleur jaune clair |
| Crospovidone | Polyplasdone® XL-10 | 0,15 | La nicotine pénétre mais ne s'étale pas ; jaunissement important |
| Silicone résine /POE Laurylether 98/2 | DC 9506® | 0,60 | Poudre qui mouille bien, mais tendance à suinter |

Comme on peut le constater, la plupart des supports testés combinés à la nicotine présentent un grand nombre d'inconvénients : impossibilité d'étalement, suintement, formation d'agrégats, de grappes ou de grumeaux, qui les rendent inutilisables.

Les polymères réticulés MMA/GDMA (polymère réticulé de méthylméthacrylate et d'éthylèneglycol diméthacrylate) permettent quant à eux une bonne adsorption de la nicotine et présentent après adsorption une bonne apparence.

### Exemple 1B : Test de capacité de relarguage des supports d'adsorption (tableau 2) :

Les supports d'adsorption sont chargés en nicotine à 50% ou 80% de leur capacité maximum d'adsorption déterminée précédemment (cf. tableau 1 précédent). Puis on effectue un test de lavage de manière à déterminer leur capacité de relargage exprimée en % de nicotine libérée par rapport à la quantité de nicotine introduite.

Les résultats suivants ont été obtenus:

**Tableau 2 :**

| Type de support | % charge | % parti au lavage |
|---|---|---|
| Silice (Silica shells) | 80% | 90 |
| | 50% | 96 |
| Polymère réticulé MMA/GDMA (Microsponge ®) | 80% | 79 |
| | 50% | 69 |
| Polyuréthane/silice (Microsphères BPD800®) | 80% | 91 |
| | 50% | 84 |
| Crospovidone (Polyplasdone® XL-10) | 80% | 99 |
| | 50% | 89 |
| Silicone résine /POE Laurylether 98/2 (DC9506®) | 80% | 72 |
| | 50% | 69 |

Le test de lavage est réalisé sur 200 mg de microparticules chargées, le rinçage est effectué avec 1 ml d'eau sur une durée de 10s d'agitation, suivi d'une filtration.

Ces résultats montrent que les polymères réticulés MMA/GDMA et la résine silicone DC9506® retiennent plus la nicotine par une meilleure adsorption que les autres supports.

### Exemple 1C : Tests de dispersion des supports d'adsorption

Des essais de dispersion de supports microporeux sont réalisés dans des solvants constituant la phase majoritaire des mélanges adhésifs avant enduction. Ces tests de dispersion permettent de vérifier la faisabilité technique de mélanges adhésifs.

Ces tests sont réalisés selon le mode opératoire suivant :
- Dans un flacon verre de 9mL, pesée du support microporeux, entre 0,2 et 1,25g selon sa densité,
- ajout du solvant (acétate d'ethyle ou heptane) de manière à respecter un ratio massique solvant / support microporeux égal environ à trois,
- mélange à la spatule et observation immédiate du mélange ; le mélange est également observé après 48h de repos.

Les résultats sont reportés dans le tableau 3 :

**Tableau 3 :**

| Type de support | Observations dans le cas de l'acétate d'éthyle | Observations dans le cas de l'heptane |
|---|---|---|
| Polyuréthane/silice (Microsphères BPD800®) | Suspension liquide | Suspension liquide |
| Crospovidone (Polyplasdone® XL-10) | Suspension liquide | Suspension liquide |
| Polymère réticulé MMA/GDMA (Microsponge ®) | Suspension pâteuse | Suspension liquide |
| Silice (Silica shells) | Support reste sec | Support reste sec |
| Silice pyrogénée densifiée (Aerosil® 200VV) | Changement d'aspect du support : présence d'agglomérats translucides | Support reste sec |
| Silice (MSS-500/3H4®) | Changement d'aspect du support : présence d'agglomérats translucides | Changement d'aspect du support : formation d'un gel |
| Silicone résine /POE Laurylether 98/2 (DC9506®) | Changement d'aspect du support : formation d'une sorte de gel translucide | Changement d'aspect du support : formation d'une sorte de gomme |

Les supports les plus intéressants à l'issue de ces essais de dispersion dans des solvants sont les supports restant en suspension soit liquide, soit pâteuse : les polymères réticulés MMA/GDMA (Microsponge ®), le BPD800 ou le Polyplasdone XL10.

De façon surprenante, seul le support microporeux MMA/GDMA réticulé permet d'obtenir des caractéristiques intéressantes sur l'ensemble des 3 tests de caractérisation effectués. Les polymères microporeux MMA/GDMA réticulés sont donc, de façon inattendue, un support particulièrement bien adapté à la nicotine (charge, relarguage, compatibilité).

Exemple 2 : Procédé de préparation d'une composition transdermique monocouche contenant de la nicotine et un polymère microporeux de type Microsponge®:
Les préparations selon l'invention sont réalisées de la manière suivante :
L'adhésif, puis la nicotine, le polymère microporeux et optionnellement le BHT sont introduits dans une cuve de mélangeur. Les paramètres d'agitation (temps et vitesse) sont fixés de manière à éviter la formation de grumeaux et à obtenir un mélange homogène et dépendent notamment de la taille du réacteur. Après un repos de 24 heures, on enduit la masse adhésive sur un film siliconé à raison de (150 ± 5)g/m² avant de procéder à un séchage à 45°C, destiné à évaporer les solvants de l'adhésif, suivi d'un transfert de la matrice sur un support polyester transparent. Le produit alors obtenu est découpé aux formats désirés.

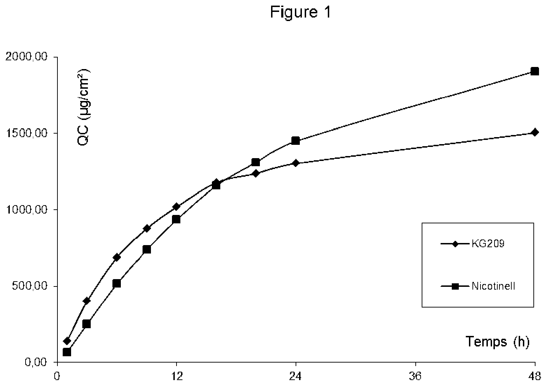

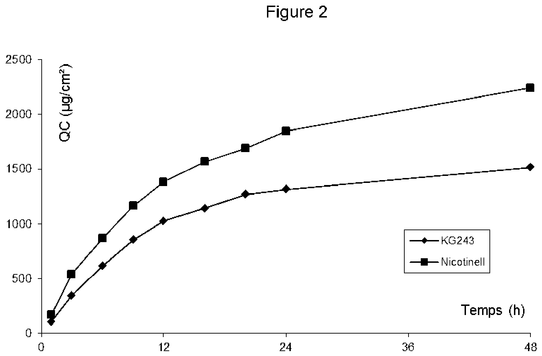

### Exemple 3 : Tests de perméation (Figures 1 et 2) :

Les compositions A et B suivantes sont obtenues selon le procédé de l'exemple 2 :

**Tableau 4**

| | KG209 | KG243 |
|---|---|---|
| Composition | A | B |
| Nicotine base | 11,2% | 11,2% |
| Polyméthyl méthacrylate/éthylène glycol diméthacrylate copolymère (Microsponge ®) | | 8,1% |
| Adhésif silicone | 9,0% | 8,1% |
| Acrylate-acétate de vinyle copolymère | 79,8% | 72,6% |

On a effectué *in vitro* une cinétique de perméation sur peau abdominale de souris nude disposée à la surface d'une cellule en verre (« cellule de Franz »). Les résultats obtenus expriment les quantités cumulées QC de 0 à 48h en µg/cm² comparatives entre une préparation ne contenant pas de polymère microporeux (formule A) et le produit de référence (Nicotinell®)(Fig.1).

| QC | | | | | | | | | |
|---|---|---|---|---|---|---|---|---|---|
| | 1 | 3 | 6 | 9 | 12 | 16 | 20 | 24 | 48 |
| KG209 | 140,47 | 399,50 | 687,86 | 876,30 | 1018,01 | 1176,65 | 1235,77 | 1302,11 | 1504,50 |
| Nicotinell | 67,47 | 249,09 | 514,86 | 737,75 | 933,88 | 1156,95 | 1310,09 | 1449,56 | 1908,67 |

La même préparation est ensuite réalisée mais contenant une quantité de polymère microporeux de type polymère réticulé MMA/GDMA (Microsponge®) selon l'invention (formule B). On a effectué *in vitro* une cinétique de perméation sur peau abdominale de souris nude disposée à la surface d'une cellule en verre (« cellule de Franz »). Les résultats obtenus expriment les quantités cumulées QC de 0 à 48h en µg/cm² comparatives entre cette préparation selon l'invention (formule B) et le produit de référence (Nicotinell® constitué d'un dépôt gélifié de nicotine sur une couche adhésive) (Fig.2).

| QC | | | | | | | | | |
|---|---|---|---|---|---|---|---|---|---|
| | 1 | 3 | 6 | 9 | 12 | 16 | 20 | 24 | 48 |
| KG243 | 106,06 | 347,84 | 614,78 | 854,52 | 1024,88 | 1143,59 | 1268,95 | 1313,79 | 1515,68 |
| Nicotinell | 171,45 | 540,27 | 869,15 | 1166,56 | 1382,27 | 1567,33 | 1690,11 | 1849,09 | 2242,83 |

Ces résultats montrent comment la présence de support microporeux de type polymère réticulé MMA/GDMA (Microsponge®) est capable de contrôler la cinétique de perméation de la nicotine à partir du patch matriciel selon l'invention. En particulier, la présence de microsphères permet de réduire la quantité de nicotine libérée dans les douze premières heures (effet « burst » observé figure 1 pour la composition KG209 (courbe qui croise celle de Nicotinell®) non observé pour la composition selon l'invention (figure 2). La réduction de l'effet « burst » est très important dans le cas de la nicotine pour éviter le relargage trop rapide d'une trop grande quantité de nicotine qui pourrait s'avérer irritant voir toxique.

L'expérience a été répétée avec une formule C différant de la formule B en ce que les 8,1% de polymère réticulé MMA/GDMA (Microsponge®) ont été remplacés par 8,1% de lauryl méthacrylate/glycol diméthylméthacrylate crosspolymer (Polytrap®). Les résultats de cinétique de pérméation obtenus avec cette formule C ont été comparés avec ceux de la formule B et ceux de Nicotinell obtenus lors de la même expérience afin que les résultats puissent être directement comparés.

| | | | | | |
|---|---|---|---|---|---|
| Temps (heures) | 0,5 | 1 | 1,5 | 3 | 6 |
| POLYTRAP | 7,6 | 32,7 | 63,9 | 156,5 | 293,5 |
| MICROSPONGE | 6,2 | 28,3 | 55,8 | 139 | 266,4 |
| | | | | | |
| 12 | | 18 | | 24 | |
| 461,8 | | 547,3 | | 599,3 | |
| 438,9 | | 530,5 | | 587,6 | |

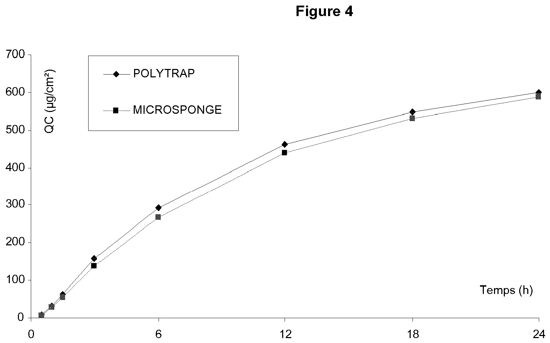

Comme on peut le constater dans le tableau ci-dessus et dans la Figure 4 annexée, les résultats obtenus sont comparables lors de l'utilisation de Microsponge® ou de Polytrap®.

Les exemples suivants illustrent l'influence du support microporeux sur les propriétés adhésives et cohésives de la matrice adhésive contenant de la nicotine.

On a obtenu les compositions monocouches adhésives C, D et E suivantes, en suivant le protocole de l'Exemple 2 :

**Tableau 5 :**

| | SME416 | SME417 | SME418 |
|---|---|---|---|
| Composition | C | D | E |
| Nicotine base | 0% | 5.0% | 5.0% |
| Polyméthyle méthacrylate/éthylene glycol diméthacrylate copolymère (Microsponge ®) | | 4.1% | |
| Butylhydroxytoluène | | 0.1% | 0.1% |
| Acrylate-acétate de vinyle copolymère | 100.0% | 90.8% | 94.9% |

Ces compositions sont alors caractérisées par des tests d'adhésion, respectivement un test de résistance au cisaillement et un test d'adhésion instantanée. Le test de résistance au cisaillement (« shear test ») consiste à mesurer la capacité d'un adhésif ou d'une formulation auto adhésive comme un patch, à résister à une force statique appliquée dans le même plan. Le critère mesuré est celui du temps nécessaire pour séparer par glissement le matériau testé d'une surface standard comme une plaque d'acier. Plus ce temps sera important, plus la cohésion sera forte. Le test d'adhésion instantanée (« tack test »), consiste à mesurer la force nécessaire pour décoller, à une vitesse donnée, un adhésif ou une formulation auto adhésive comme un patch, d'une surface standard avec laquelle il a été mis en contact sous l'effet d'une faible pression (par réalisation d'une boucle au contact d'une sonde). Le critère mesuré est une force exprimée en Newton. Plus cette force est élevée, plus le l'adhésion immédiate est élevée.

Les résultats suivants ont été obtenus (Tableau 4):

**Tableau 6 :**

| | Adhésion instantanée Force max (Newton) | Temps de résistance au cisaillement |
|---|---|---|
| Composition C | 19.2 | Plus de 168 heures |
| Composition D | 16.6 | 112 heures |
| Composition E | 23.1 | 86 heures |

On montre ainsi que l'ajout de nicotine, par effet plastifiant, au sein d'un adhésif diminue la cohésion du système (diminution du temps de résistance au cisaillement) et augmente son adhésion instantanée (comparatif entre les compositions C et E). L'ajout de microparticules poreuses de type polymère réticulé MMA/GDMA (Microsponge®) permet d'améliorer la cohésion du système et de réduire l'agressivité de l'adhésion instantanée (comparatif des compositions D et E). L'ajout de microparticules poreuses de type polymère réticulé MMA/GDMA (Microsponge®) permet donc de façon inattendue de réduire l'effet plastifiant de la nicotine.

### Exemple 5 : Influence de l'adhésif sur la libération de nicotine

Les exemples suivants illustrent pour les préparations de compositions selon l'invention, l'influence l'adhésif sur la libération de nicotine.

On a obtenu les compositions monocouches adhésives suivantes, en suivant le protocole de l'Exemple 2 :

**Tableau 7:**

| | KG427 | KG428 | KG429 |
|---|---|---|---|
| Nicotine base | 10,0% | 10,0% | 10% |
| Polymethyl methacrylate/ethylene glycol dimethacrylate copolymère | 8,3% | 8,3% | 8,3% |
| Butylhydroxytoluène | 0,1% | 0,1% | 0,1% |
| Adhésif acrylate-acétate de vinyle auto-réticulant (Duro-Tak® 2052) | 81,6% | | |
| Adhésif acrylique non-réticulé (Duro-Tak® 9088) | | 81,6% | |
| Adhésif acrylate-acétate de vinyle auto-réticulant (Duro-Tak® 2196) | | | 81,6% |

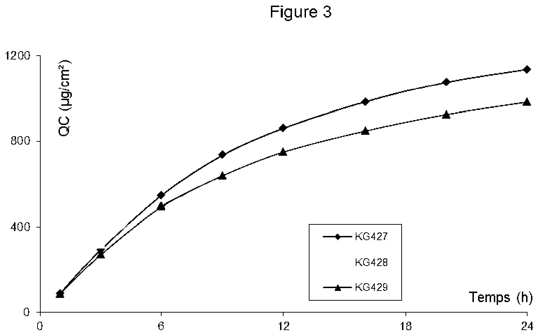

Le test de perméation *in vitro* a été conduit selon la description faite dans l'exemple 3 précédent et les résultats obtenus sont ceux de la Figure 3.

| QC | 1 | 3 | 6 | 9 | 12 | 16 | 20 | 24 |
|---|---|---|---|---|---|---|---|---|
| KG427 | 90 | 294 | 547 | 735 | 860 | 983 | 1076 | 1137 |
| KG428 | 118 | 319 | 501 | 613 | 687 | 776 | 842 | 888 |
| KG429 | 87 | 271 | 493 | 639 | 749 | 847 | 924 | 982 |

L'utilisation de microparticules poreuses de type polymère réticulé MMA/GDMA (Microsponge®) permet d'obtenir pour un patch un profil cinétique *in vitro* semblable quel que soit l'adhésif utilisé.

## Revendications

1. Dispositif transdermique auto-adhésif, **caractérisé en ce qu'**il comprend l'association d'une couche support et d'une couche matricielle auto-adhésive et un film protecteur détachable, ladite couche matricielle auto-adhésive comprenant, par rapport au poids total de la couche matricielle auto-adhésive :
a. 65 à 93 % en poids d'au moins un polymère auto-adhésif choisi dans le groupe comprenant les polymères de type acrylique ou acrylate, les polymères de type silicone, les polymères de type acétate de vinyle, les gommes naturelles ou synthétiques, leurs copolymères et leurs mélanges ;
b. 2 à 15% en poids d'au moins un polymère solide microporeux contenant de la nicotine, ledit polymère étant constitué d'unités monomères de laurylméthacrylate et de glycol diméthacrylate ; et
c. 5 à 20% en poids de nicotine.

2. Dispositif selon la revendication 1, **caractérisé en ce que** le polymère auto-adhésif est de type acrylique ou acrylate et est constitué par des monomères sélectionnés dans le groupe comprenant l'acétate de vinyle, le 2-éthylhexyl acrylate, l'acrylate de butyle, l'acide acrylique, le méthyl méthacrylate, le méthyl acrylate, le ter octyl acryl amide, le 2-hydroxy éthyl acrylate, le glycidyl méthacrylate, ou leurs mélanges.

3. Dispositif selon la revendication 2, **caractérisé en ce que** les monomères sont choisis dans le groupe comprenant l'acide acrylique, l'acrylate de butyle, le 2-éthylhéxyl acrylate, l'acétate de vinyle, ou leurs mélanges.

4. Dispositif selon l'une quelconque des revendications 1 à 3, **caractérisé en ce que** ledit polymère solide microporeux se présente sous la forme de microparticules de taille moyenne comprise entre 1 µm et 100 µm, préférentiellement entre 5 µm et 50 µm, encore préférentiellement entre 15 µm et 30 µm.

5. Dispositif selon l'une quelconque des revendications 1 à 4, **caractérisé en ce que** ledit polymère solide microporeux à un volume poreux total compris entre 0,5 ml/g et 3,0 ml/g, préférentiellement entre 1 ml/g et 2 ml/g.

6. Dispositif selon l'une quelconque des revendications 1 à 5, **caractérisé en ce que** le dit polymère solide microporeux à un volume poreux granulaire compris entre 0,2 ml/g et 3,0 ml/g, préférentiellement entre 0,5 ml/g et 1,5 ml/g.

7. Dispositif selon l'une des revendications 1 à 6, **caractérisé en ce que** le polymère solide microporeux est distribué de manière homogène au sein de la masse de polymère auto-adhésif.

8. Dispositif selon l'une des revendications 1 à 7, **caractérisé en ce que** le principe actif est adsorbé de manière homogène dans la masse de polymère solide microporeux.

9. Dispositif selon l'une quelconque des revendications 1 à 8, **caractérisé en ce que** le grammage de la couche matricielle autoadhésive est compris entre 50 et 300 g/m², préférentiellement entre 100 et 200 g/m².

10. Dispositif selon l'une quelconque des revendications 1 à 9, **caractérisé en ce que** la couche matricielle auto-adhésive comprend au moins un agent antioxydant choisi dans le groupe comprenant le butylhydroxytoluène (BHT), le butyl hydroxy anisole (BHA), le palmitate d'ascorbyle, l'alpha tocophérol et ses esters, l'acide citrique, le gallate de propyle et leurs mélanges.

11. Dispositif selon la revendication 10, **caractérisé en ce que** l'agent antioxydant est présent dans ladite couche matricielle auto-adhésive, par rapport au poids total de ladite couche matricielle auto-adhésive, dans une proportion comprise entre 0,01 et 1% en poids.

12. Dispositif selon l'une quelconque des revendications 1 à 11, **caractérisé par** un flux moyen de nicotine à partir de la couche matricielle est compris entre 10 µg/cm²/h et 50 µg/cm²/h, pendant un intervalle de temps compris entre 1 et 24h.

13. Procédé de préparation d'un dispositif transdermique selon l'une quelconque des revendications 1 à 12, **caractérisé en ce qu'**il comprend les étapes suivantes :
a. Mélanger le polymère auto-adhésif, le polymère solide microporeux, la nicotine et optionnellement l'agent oxydant jusqu'à obtention d'un mélange homogène ;
b. Enduire le mélange homogène obtenu à l'étape a. sur un film protecteur détachable;
c. Sécher progressivement le mélange enduit;
d. Contrecoller le produit obtenu à l'étape c. sur une couche support ; et
e. Optionnellement, découper le produit obtenu à l'étape d. de manière à obtenir des dispositifs transdermiques ou patchs de taille désirée.

14. Dispositif selon l'une quelconque des revendications 1 à 12, pour son utilisation en tant que médicament.

15. Dispositif selon la revendication 14, pour son utilisation dans le traitement de la dépendance à la nicotine.

16. Dispositif selon la revendication 14, pour son utilisation dans le traitement des maladies neurodégénératives.

## Patentansprüche

1. Transdermale selbstklebende Vorrichtung, **dadurch gekennzeichnet, dass** sie den Verbund einer Trageschicht und einer selbstklebenden Matrixschicht und einer ablösbaren Schutzfolie umfasst, wobei die besagte selbstklebende Matrixschicht, bezogen auf das Gesamtgewicht der selbstklebenden Matrixschicht, Folgendes umfasst:
a. 65 bis 93 Gew.-% mindestens eines selbstklebenden Polymers, das aus der Gruppe umfassend Polymere vom Acryl- oder Acrylat-Typ, Polymere vom Silikon-Typ, Polymere vom Vinylacetat-Typ, Natur- und synthetische Gummi, deren Copolymere und deren Gemische ausgewählt ist;
b. 2 bis 15 Gew.-% mindestens eines mikroporösen festen Polymers, Nikotin enthaltend, wobei das genannte Polymer sich aus Laurylmethacrylat- und Glycol-Dimethacrylat-Monomereinheiten zusammensetzt; und
c. 5 bis 20 Gew.-% Nikotin.

2. Vorrichtung nach Anspruch 1, **dadurch gekennzeichnet, dass** das selbstklebende Polymer vom Acryl- oder Acrylat-Typ ist und sich aus Monomeren zusammensetzt, die aus der Gruppe umfassend Vinylacetat, 2-Ethylhexylacrylat, Butylacrylat, Acrylsäure, Methylmethacrylat, Methylacrylat, tert.-Octylacrylamid, 2-Hydroxyethylacrylat, Glycidylmethacrylat oder deren Gemische ausgewählt sind.

3. Vorrichtung nach Anspruch 2, **dadurch gekennzeichnet, dass** die Monomere aus der Gruppe umfassend Acrylsäure, Butylacrylat, 2-Ethylhexylacrylat, Vinylacetat oder deren Gemische ausgewählt sind.

4. Vorrichtung nach einem der Ansprüche 1 bis 3, **dadurch gekennzeichnet, dass** das besagte mikroporöse feste Polymer in der Form von Mikropartikeln mit einer durchschnittlichen Größe zwischen 1 µm und 100 µm, vorzugsweise zwischen 5 µm und 50 µm, mehr bevorzugt zwischen 15 µm und 30 µm vorliegt.

5. Vorrichtung nach einem der Ansprüche 1 bis 4, **dadurch gekennzeichnet, dass** das besagte mikroporöse feste Polymer ein Gesamtporenvolumen zwischen 0,5 ml/g und 3,0 ml/g, vorzugsweise zwischen 1 ml/g und 2 ml/g aufweist.

6. Vorrichtung nach einem der Ansprüche 1 bis 5, **dadurch gekennzeichnet, dass** das genannte mikroporöse feste Polymer ein granuläres Porenvolumen zwischen 0,2 ml/g und 3,0 ml/g, vorzugsweise zwischen 0,5 ml/g und 1,5 ml/g aufweist.

7. Vorrichtung nach einem der Ansprüche 1 bis 6, **dadurch gekennzeichnet, dass** das mikroporöse feste Polymer homogen in der Masse des selbstklebenden Polymers verteilt ist.

8. Vorrichtung nach einem der Ansprüche 1 bis 7, **dadurch gekennzeichnet, dass** der Wirkstoff homogen in der Masse des mikroporösen festen Polymers adsorbiert ist.

9. Vorrichtung nach einem der Ansprüche 1 bis 8, **dadurch gekennzeichnet, dass** das Flächengewicht der selbstklebenden Matrixschicht zwischen 50 und 300 g/m², vorzugsweise zwischen 100 und 200 g/m² liegt.

10. Vorrichtung nach einem der Ansprüche 1 bis 9, **dadurch gekennzeichnet, dass** die selbstklebende Matrixschicht mindestens ein Antioxidationsmittel umfasst, das aus der Gruppe umfassend Butylhydroxytoluol (BHT), Butylhydroxyanisol (BHA), Ascorbylpalmitat, alpha-Tocopherol und seine Ester, Citronensäure, Propylgallat und deren Gemische ausgewählt ist.

11. Vorrichtung nach Anspruch 10, **dadurch gekennzeichnet, dass** das Antioxidationsmittel in der besagten selbstklebenden Matrixschicht, bezogen auf das Gesamtgewicht der besagten selbstklebenden Matrixschicht, in einem Anteil zwischen 0,01 und 1 Gew.-% vorhanden ist.

12. Vorrichtung nach einem der Ansprüche 1 bis 11, **dadurch gekennzeichnet, dass** ein durchschnittlicher Nikotinfluss von der Matrixschicht zwischen 10 µg/cm²/h und 50 µg/cm²/h während eines Zeitraums zwischen 1 und 24 h liegt.

13. Verfahren zur Herstellung einer transdermalen Vorrichtung nach einem der Ansprüche 1 bis 12, **dadurch gekennzeichnet, dass** es die folgenden Schritte umfasst:
a. Mischen des selbstklebenden Polymers, des mikroporösen festen Polymers, des Nikotins und gegebenenfalls des Antioxidationsmittels bis zum Erhalten eines homogenen Gemischs;
b. Überziehen einer ablösbaren Schutzfolie mit dem im Schritt a. erhaltenen homogenen Gemisch;
c. allmähliches Trocknen des überzogenen Gemischs;
d. Laminieren des im Schritt c. erhaltenen Produkts auf eine Trageschicht; und
e. gegebenenfalls Schneiden des im Schritt d. erhaltenen Produkts, um transdermale Vorrichtungen oder Pflaster mit der gewünschten Größe zu erhalten.

14. Vorrichtung nach einem der Ansprüche 1 bis 12 zu deren Verwendung als Arzneimittel.

15. Vorrichtung nach Anspruch 14 zu deren Verwendung bei der Behandlung von Nikotinabhängigkeit.

16. Vorrichtung nach Anspruch 14 zu deren Verwendung bei der Behandlung von neurodegenerativen Erkrankungen.

## Claims

1. Self-adhesive transdermal device, **characterised in that** it includes the association of a support layer and a self-adhesive matrix layer and a detachable protective film, said self-adhesive matrix layer comprising, in relation to the total weight of the self-adhesive matrix layer:
a. 65 to 93% by weight of at least one self-adhesive polymer selected from the group comprising polymers of the acrylic or acrylate type, polymers of the silicone type, polymers of the vinyl acetate type, natural or synthetic gums, copolymers thereof and mixtures thereof;
b. 2 to 15% by weight of at least one microporous solid polymer including nicotine, said polymer being comprised of monomer units of lauryl methacrylate and glycol dimethacrylate; and
c. 5 to 20% by weight of nicotine.

2. Device according to claim 1, **characterised in that** the self-adhesive polymer is of the acrylic or acrylate type and being comprised of monomers selected from the group comprising vinyl acetate, 2-ethylhexyl acrylate, butyl acrylate, acrylic acid, methyl methacrylate, methyl acrylate, tert-octyl acrylamide, 2-hydroxy ethyl acrylate, glycidyl methacrylate, or mixtures thereof.

3. Device according to claim 2, **characterised in that** the monomers are selected from the group comprising acrylic acid, butyl acrylate, 2-ethylhexyl acrylate, vinyl acetate, or mixtures thereof.

4. Device according to any of claims 1 to 3, **characterised in that** said microporous solid polymer is provided in the form of microparticles of average size comprised between 1 µm and 100 µm, preferably between 5 µm and 50 µm, more preferably between 15 µm and 30 µm.

5. Device according to any of claims 1 to 4, **characterised in that** said microporous solid polymer has a total pore volume comprised between 0.5 ml/g and 3.0 ml/g, preferably between 1 ml/g and 2 ml/g.

6. Device according to any of claims 1 to 5, **characterised in that** said microporous solid polymer has a granular pore volume comprised between 0.2 ml/g and 3.0 ml/g, preferably between 0.5 ml/g and 1.5 ml/g.

7. Device according to any of claims 1 to 6, **characterised in that** the microporous solid polymer is distributed homogeneously within the self-adhesive polymer mass.

8. Device according to any of claims 1 to 7, **characterised in that** the active ingredient is adsorbed homogeneously into the microporous solid polymer mass.

9. Device according to any one of claims 1 to 8, **characterised in that** the basis weight of the self-adhesive matrix layer is comprised between 50 and 300 g/m², preferably between 100 and 200 g/m².

10. Device according to any one of claims 1 to 9, **characterised in that** the self-adhesive matrix layer includes at least one antioxidant agent selected from the group comprising butylhydroxytoluene (BHT), butyl hydroxy anisole (BHA), ascorbyl palmitate, alpha tocopherol and esters thereof, citric acid, propyl gallate and mixtures thereof.

11. Device according to claim 10, **characterised in that** the antioxidant agent is present in said self-adhesive matrix layer, in relation to the total weight of said self-adhesive matrix layer, in a proportion comprised between 0.01 and 1% by weight.

12. Device according to any of claims 1 to 11, **characterised by** an average nicotine delivery rate from the matrix layer comprised between 10 µg/cm²/h and 50 µg/cm²/h, during a time interval comprised between 1 and 24 hours.

13. Method for preparing a transdermal device according to any one of claims 1 to 12, **characterised in that** it comprises the following steps:
a. mixing the self-adhesive polymer, the microporous solid polymer, nicotine and optionally the oxidising agent until a homogeneous mixture is obtained;
b. spreading the homogeneous mixture obtained in step a on a detachable protective film;
c. gradually drying the spread mixture;
d. laminating the product obtained in step c onto a support layer; and
e. optionally cutting out the product obtained in step d in such a way as to obtain transdermal devices or patches of the desired size.

14. Device according to any one of claims 1 to 12, for use as a drug.

15. Device according to claim 14, for use in the treatment of nicotine addiction.

16. Device according to claim 14, for use in the treatment of neurodegenerative diseases.
